# EUROPÄISCHE PATENTSCHRIFT

(11) **EP 0 222 146 B1**
(45) Veröffentlichungstag und Bekanntmachung des Hinweises auf die Patenterteilung: **16.02.1994**
(21) Anmeldenummer: 86113784.2
(22) Anmeldetag: 04.10.1986
(51) Int. Cl.: G01N 33/547, G01N 33/551, B01J 20/10

(54) **Selektives Adsorbens zur Bindung von Immunkomplexen**
Selective adsorbent for binding immunocouplers
Adsorbant sélectif pour la fixation de complexes immunogènes

(30) Priorität: 16.10.1985 DD 281795; 16.10.1985 DD 281794; 16.10.1985 DD 281793
(43) Veröffentlichungstag der Anmeldung: 20.05.1987
(73) Patentinhaber: Fresenius AG, 61440 Oberursel (DE)
(72) Erfinder: Hiepe, Falk, Dr., DD-1170 Berlin (DD); Schössler, Werner, Dr., DD-1297 Schwanebeck (DD); Apostoloff, Emil, Prof.Dr., DD-1105 Berlin (DD); Precht, Klaus, Prof.Dr., DD-1080 Berlin (DD); Heymann, Stephan, Dipl.Phys.Dr., DD-1058 Berlin (DD); Coupek, Jiri, Dipl.Ing.Dr., CS-14400 Prag (CS); Becker, Manfred, Dr., DD-1100 Berlin (DD)
(74) Vertreter: Eitle, Werner, Dipl.-Ing.

(56) Entgegenhaltungen:
- DD-A- 225 795
- DE-A- 2 501 831
- DE-A- 2 938 636
- DE-B- 2 042 976
- US-A- 4 283 383
- US-E- 29 474
- BIOLOGICAL ABSTRACTS, Teil 81, 1986, Philadelphia, PA (US); Y. GAZITT et al., Nr. 81013978#
- CHEMICAL ABSTRACTS, Teil 95, Nr. 19, 09 November 1981, Columbus, OH (US); J.J. GIBBONS et al., Seite 538, Nr. 166857w#

## Beschreibung

Die Erfindung betrifft die Verwendung von an eine Festphase kovalent gebundenem C1q zur Herstellung eines Therapeutikums zur Bindung von Immunkomplexen. Die Erfindung ist in der pharmazeutischen Industrie und der Medizin anwendbar.

Es gilt als gesichert, daß den in Körperflüssigkeiten, z.B. im Blut, zirkulierenden Antigen-Antikörper-Komplexen (Immunkomplexe) eine wesentliche pathogenetische Bedeutung bei einer Vielzahl von Erkrankungen (u.a. Autoimmunerkrankungen wie Lupus erythematodes visceralis und Rheumatoid-Arthritis, Transplantatabstoßung, Infektionen, möglicherweise auch bei Neoplasien) zukommt. US-A-4 283 383 beschreibt die Verwendung von kovalent an Festphasensubstrate gebundenem C1q zur Analyse biologischer Flüssigkeiten. BBA 670,146-149 (1981) beschreibt die Verwendung von C1q-Sepharose^{R} Säulen zur Quantifizierung von Immunkomplexen. Zirkulierende Immunkomplexe können sich an den Gefäßwänden ablagern und über verschiedene Wirkungsmechanismen entzündliche Reaktionen in den jeweils disponierten Organen mit den entsprechenden Symptomen (u.a. Vasculitis, Arthritis, Nephritis, Serositis, Karditis, Cerebritis, Anämie, Leukopenie, Thrombopenie) induzieren. Insbesondere bei Autoimmunerkrankungen korreliert die Immunkomplexkonzentration im Serum/Plasma oder anderen Körperflüssigkeiten mit der Aktivität der Erkrankung. Deshalb erscheint es sinnvoll, den Pathomechanismus dieser Erkrankungen durch Entfernung zirkulierender Immunkomplexe aus den Körperflüssigkeiten zu beeinflussen, um dadurch das Fortschreiten der oben erwähnten Erkrankungen und unerwünschte Immunreaktionen zu verhindern, die Krankheitsaktivität zu mindern, die damit verbundenen Symptome zu lindern bzw. zu beseitigen. Die Erfolge der Plasmapheresebehandlung werden u.a. auf die Beseitigung bzw. Reduzierung der pathogenetisch bedeutsamen Immunkomplexe zurückgeführt [vgl. E. APOSTOLOFF et al., Dt. Gesundheitsw. 34, 64 (1979); H.F. PARRY et al., Ann. Rheum. Dis. 40, 224-228 (1981); T. SHARON et al., Plasma Ther. Transfus. Technol. 3, 163-169 (1982); M. VALBONESI et al., Int. J. Artif. Organs 4, 234-236 (1981); J.V. JONES et al., Arthritis Rheum. 24, 1113-1120 (1981)].

Nachteile dieser Methode sind das nicht selektive Entfernen aller Plasmabestandteile und die dadurch erforderliche Substitution größerer Mengen Fremdplasma oder Humanalbumin, die auch aus Kapazitäts- und ökonomischen Gründen limitierend sein können. Aus den genannten Gründen existieren seit einigen Jahren Bemühungen um die Entwicklung von Adsorbentien, die eine selektive Entfernung zirkulierender Immunkomplexe aus Körperflüssigkeiten erlauben, hierbei wurden im wesentlichen folgende Wege beschritten:
- Staphylokokken-Protein A, an einen unlöslichen Träger fixiert (fixierte Staphylokokken, Protein A-Sepharose) [vgl. D.S. TERMEN et al., J. Immunol. 124, 798 (1980); New England J. Med. 305, 1195; E. BEHM u. H. KLINKMANN, in Plasma Fractionation, S. 254-265 (Karger, Basel 1983)]
- Poröses Harz vom Akrylsäureester-Typ [z.B. XAS-7, geliefert von Rohm und Hass Co., USA; vgl. T. AGISHI, Zinko Zoki 9, 264 (1980)]
- Kationenaustauschglied wie Carboxymethylcellusose [vgl. L. D. JOHNSON et al., Can. J. Biochem. 42, 795 (1964)]
- Adsorbens, enthaltend eine Oberfläche und mit der Oberfläche verbunden wenigstens ein hydrophobes Glied mit 6 - 700 Kohlenstoff-Atomen und wenigstens eine negative Ladung erzeugendes Glied [T. KURODA et al., japanisches Patent J. P. P58-59197, P58-59199]
Diese Adsorbentien haben jedoch verschiedene Nachteile: Protein A ist in der Lage, Immunkomplexe zu binden, jedoch nicht ausreichend selektiv, denn es bindet ebenfalls nicht immunkomplexgebundenes IgG. Außerdem ist Protein A ein Fremdprotein, mit den ihm anhaftenden Nachteilen beim Einsatz des Adsorbens, wenn es in Berührung mit den Körperflüssigkeiten gehalten wird. Die Herstellungskosten für Protein A sind sehr hoch. Die Verwendung inaktivierter Staphylokokken als "natürliche" Träger des Proteins A ermöglicht den Einsatz des Proteins A ohne seine weitere Reinigung, bringt aber zusätzliche Probleme bei Berührung des Plasmas mit den inaktivierten Bakterien (z.B. Freisetzung von Pyrogenen) mit sich. Weiterhin sollen Protein-A-Träger das Komplementsystem aktivieren. Das poröse Harz vom Acrylsäureester-Typ und Carboxymethylcellulose besitzen nur ungenügendes Adsorptionsvermögen und unzureichende Adsorptionsspezifität (u. a. wird Albumin aus dem Plasma adsorbiert). Das aus einem hydrophoben und einem eine negative Ladung enthaltende Glied bestehende Adsorbens ist ebenfalls für die Adsorption von Immunkomplexen nicht ausreichend spezifisch.

Hier will die Erfindung Abhilfe schaffen. Der Erfindung liegt deshalb die Aufgabe zugrunde, zirkulierende Immunkomplexe selektiv, einfach und schonend aus Körperflüssigkeiten unter Nutzung sterilisierbarer Träger zu entfernen.

Gegenstand der Erfindung ist die Verwendung gemaß Patentanspruch 1.

Zweckmäßige Ausführungen davon sind Gegenstand der Ansprüche 2 bis 9.

Die erfindungsgemäß verwendeten Träger lassen sich einfach, schnell und reproduzierbar zwecks Wiederverwendung regenerieren. Die erfindungsgemäße Lösung der Aufgabe erfolgt dadurch, daß das Protein C1q - eine Untereinheit der ersten Komplementkomponente - biospezifisch an beschichtete Träger, vorzugsweise makroporöse sphärische Copolymere von 2-Hydroxyethylmethacrylat mit Ethylenglykoldimethacrylat oder flächenförmige Copolymere der gleichen chemischen Struktur oder an zellulosehaltige Materialien, vorzugsweise Perlzellulose oder flächenförmige Zelluloseträger, über eine Brückenbildung oder an siliziumdioxidhaltige Träger, vorzugsweise poröses Glas, Glasfasern oder flächenförmige Glasfaserfilter, über Silanhaftmittel oder an Copolymere von Methacrylamid, Methylenbisacrylamid, Glycydylether und/oder Allylglycydylether oder an andere geeignete Träger kovalent gebunden wird und dieses trägergebundene Protein C1q zur Bindung und in vivo Entfernung der zirkulierenden Immunkomplexe mittels extrakorporaler Immunadsorption eingesetzt wird, wobei die an das Protein C1q gebundenen Immunkomplexe aus den Körperflüssigkeiten durch Abtrennung der festen Phase separiert werden und die feste Phase durch Behandlung mit nicht denaturierenden Medien funktionell regeneriert wird. Das Verfahren ist weiterhin dadurch gekennzeichnet, daß das Protein C1q an oben genannte Träger, die eine hinreichend große Oberfläche und Porosität aufweisen, durch bekannte vorherige chemische Oberflächenmodifizierung des Trägers mit Epichlorhydrin bzw. Chlorameisensäureester, CNBr, Cyanurchlorid, Carbodiimiden (Übersicht: M. D. LILLY; Methods in Enzymology 44, (1976), 467) oder aber mit Silanhaftmitteln und gege benenfalls hetero- oder homobifunktionellen Reagenzien gebunden wird. Der als feste Phase dienende Träger hat eine unterschiedliche geometrische Form, wobei kugelförmige, faserförmige oder flächenförmige vernetzte Träger mit oder ohne Zusätze besonders geeignet sind. Denkbar sind aber auch Filme, Lacke und andere dünne Schichten der genannten Copolymere auf anderen inerten und biologisch verträglichen Trägermaterialien. Das Poly(hydroxyethylmethacrylat)-haltige Material hat im wesentlichen eine sphärische Struktur, wobei deren Partikelgröße, Zusammensetzung und Porosität unterschiedlich sein kann. Besonders vorteilhaft sind Partikelgrößen zwischen 100 - 400 µm und Porenweiten >50 nm. Die maximale Porenweite beträgt ca. 100 µm, wobei mit zunehmender Porenweite die zur Verfügung stehende Fläche und damit die Kapazität des Trägers immer kleiner wird.

Das zellulosehaltige Material ist im wesentlichen Perlzellulose, wobei deren Partikelgröße, Zusammensetzung, Struktur und Porosität unterschiedlich sein kann. Besonders vorteilhaft sind Partikelgrößen zwischen 100 - 400 µm und Porenweite >50 nm. Die maximale Porenweite beträgt ca. 100 µm, wobei mit zunehmender Porenweite die zur Verfügung stehende Fläche und damit die Kapazität des Trägers in analoger Art und Weise immer kleiner wird. Das Protein C1q wird an den zellulosehaltigen Träger auf an sich bekannte vorherige chemische Oberflächenmodifikation des Trägers, vorzugsweise durch Chlorameisensäureester, CNBr, Cyanurchlorid, Carbodiimiden oder aber mit Silanhaftmitteln gebunden. Der siliziumdioxidhaltige Träger hat ebenfalls eine unterschiedliche geometrische Form, wobei kugelförmige oder faserförmige Träger mit oder ohne Zusätze, die auch durch Packung, Sinterung oder Verwendung eines Bindemittels zu übergeordneten Strukturen (wir Filtern, Fritten und dergleichen) mit definierter Durchlässigkeit angeordnet sein können, besonders geeignet sind. Die siliziumdioxidhaltigen Materialien sind im wesentlichen poröse Gläser (s. hierzu F. JANOWSKI, W. HEYER; Poröse Gläser, Deutscher Verlag für Grundstoffindustrie, Leipzig 1982), die bekanntlich problemlos sterilisiert und steril gehalten werden können, wobei deren Partikelgröße, Struktur und Porosität genau festgelegt wird. Besonders vorteilhaft sind ideal sphärische Partikel (DD-WP CO3B 259 5548, DD-WP BO1 J 269 4382, DD-WP BO1 J 26 94390 mit Partikelgrößen zwischen 1 bis 1 000 µm und Porenweiten von 0,05 bis 100 µm, wobei sich die Porenweiten und die zur Verfügung stehende Fläche, die die Kapazität des Trägers entscheidend beeinflußt, reziprok proportional verhalten. Die ideal sphärische Form der Matrix hat einen entscheidenden Einfluß auf die schonenden Bedingungen des Verfahrens, da die auf die Proteine einwirkenden Scherkräfte drastisch reduziert sind. Des weiteren zeichnen sich die ideal sphärischen und abriebfesten Glaspartikel durch ihre Erosionsbeständigkeit, ihre günstigen hydrodynamischen Eigenschaften, ihre Variationsbreite der Textur und somit auch der Durchflußeigenschaften sowie durch die Abwesenheit toxischer Bestandteile aus. Insbesondere unter dem Aspekt technologischer Verfahren sind flächenförmige Träger oder Patronen mit Kornmaterial oder Membranen aus porösem Glas mit übergeordneten Strukturen bzw. flächenförmige Träger, die als Filter in geeigneten Filtrationssystemen eingesetzt werden, hervorzuheben. Hier zeichnen sich besonders Glasfasern enthaltende Filtermaterialien aufgrund ihrer mechanischen Festigkeit aus. Die nicht denaturierenden Medien für die Abspaltung der gebundenen Immunkomplexe setzen sich aus gepufferten Lösungen hoher Ionenstärke zusammen, wobei insbesondere 0,3 - 3 M NaCl-Lösungen oder aber auch chaotropische Reagenzien sowie Säuren und Laugen geeignet sind.
Die Oberflächenmodifizierung der Poly(hydroxyethylmethacrylate) zum Zwecke der Schaffung der Bedingungen für das Eingehen kovalenter Bindungen mit dem Protein C1q erfolgt auf an sich bekannte Art und Weise, vorzugsweise durch Epichlorhydrin, kann aber auch durch andere Modifizierungsverfahren, in denen CNBr, Carbodiimide oder andere hetero- oder homobifunktionelle Reagenzien eingesetzt werden, erfolgen. Die Oberflächenmodifizierung der Zellulose erfolgt auf an sich bekannte Art und Weise, vorzugsweise durch Chlorameisensäureester, kann aber auch durch andere bekannte Modifizierungsverfahren unter Verwendung von CNBr, Cyanurchlorid, Carbodiimide und Glutaraldehyd erfolgen.
Die Oberflächenmodifizierung der siliziumdioxidhaltigen Träger erfolgt durch sogenannte Silanhaftmittel wie 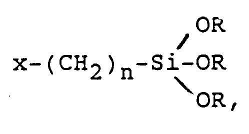
wobei R Alkylreste und x z.B. Amino-, Diazo-, Sulfhydryl- oder Nitroso-Gruppen sind, und durch Umsetzen der verbleibenden funktionellen Gruppe auf an sich bekannte Art und Weise mit homo- oder heterobifunktionellen Reagenzien oder direkte Umsetzung mit den reaktiven Gruppen des Proteins.
Der erfindungsgemäße C1q-Träger ist chemisch stabil, mikrobiell schwer angreifbar, sterilisierbar und biokompatibel. Dadurch ist die Wiederverwendbarkeit gewährleistet. Der so mit dem Protein C1q beladene Träger wird dann zur Bindung und Entfernung der zirkulierenden Immunkomplexe eingesetzt, wobei der Träger insbesondere im Durchflußverfahren in üblicher Weise (Hämoperfusion, Plasmaperfusion) für die extrakorporale Immunadsorption genutzt werden kann. Nach Ablauf der Reaktion und Entfernung des Trägers von den zu behandelnden Körperflüssigkeiten werden die gebundenen Immunkomplexe durch nicht denaturierende Medien wie z.B. 2 M NaCl-Lösung abgespalten, so daß das kovalent an den Träger gebundene Protein C1q für einen erneuten Einsatz zur Verfügung steht. Das erfindungsgemäße Verfahren nutzt die physiologische Funktion des Proteins C1q für die selektive und schonende Bindung und damit Entfernung von zirkulierenden Immunkomplexen aus Körperflüssigkeiten.

Beispiel 1: 5 g makroporöses sphärisches Copolymer von 2-Hydroxyethylmethacrylat und Ethylenglykoldimethacrylat mit einer Ausschlußgrenze von 2x10⁶ D und einer Oberfläche von ca. 70 m²/g sowie reaktiven Epoxygruppen (1,020 mMol/g Träger) (Separon^{R} Hema 1000 E) werden mit Protein C1q (1 mg/ml) in PBS (phosphate buffered saline), pH 7.4, 10 mM EDTA enthaltend, versetzt und 48 h bei 4⁰C unter leichtem Schütteln inkubiert. Die Bestimmung des gebundenen Proteins C1q erfolgt durch Differenzmessung der Ausgangslösung bzw. des Überstandes bei 280 nm (E²⁸⁰_{1%} =6,8) oder mit Hilfe der einfachen radialen Immunodiffusion mittels eines spezifischen Antiserums. Abschließend wird der Träger mit 1 M Ethanolaminlösung in PBS, pH 7.4, versetzt, um eventuell noch freie reaktive Gruppen abzublocken. Schließlich wird der Träger mit einem 0,01 M Phosphat-Puffer, pH 7.4, der 0,05 M NaCl enthält, gewaschen. Dieses trägergebundene Protein C1q wird nunmehr zur Bindung der Immunkomplexe eingesetzt. Hierzu wird der mit dem Protein C1q beladene Separon-Träger mit Heparin-Plasma, das eine erhöhte Konzentration zirkulierender Immunkomplexe enthält (Patient mit Lupus erythematodes visceralis), im batch-Verfahren versetzt und 2 h bei Raumtemperatur inkubiert. Nach Entfernung des Trägers durch Dekantieren oder Zentrifugieren wird dieser im folgenden mit 0,01 mM Phosphat-Puffer, pH 7.4, 0,05 M NaCl, gewaschen und anschließend mit 2 M NaCl in PBS, pH 7.4, regeneriert. Die Bestimmung der Immunkomplexe erfolgte mit einem Protein C1q-Festphasen-Enzymimmunoassay. Zur Ermittlung der unspezifischen Bindung an den Träger wurde die Eiweißkonzentration im spezifischen Eluat nach Lowry sowie bei 280 nm bestimmt sowie eine Immunelektrophorese, doppelte radiale Immunodiffusion mit spezifischen Antiseren und Polyacrylamidgelelektrophorese durchgeführt. Im Eluat konnten spezifisch zirkulierende Immunkomplexe nachgewiesen werden. Kontaminierende Begleitproteine waren nicht vorhanden. Eine Aktivierung des Komplementsystems konnte nicht nachgewiesen werden.

Beispiel 2: An aktiviertes Separon^{R} Hema 1000 E wird, wie in Beispiel 1 beschrieben, humanes Protein C1q gebunden. Diese Matrix wird in eine Chromatographiesäule (Durchmesser 4 mm) gefüllt und mit einem 0,01 M Phosphat-Puffer, pH 7.4, 0,05 M NaCl, gespült. Anschließend wird 2 ml Heparin-Plasma eines Patienten mit erhöhtem Immunkomplexspiegel aufgetragen und mit o.g. Puffer eluiert (Durchfluß 10 ml/h). Die Abspaltung der spezifisch gebundenen Immunkomplexe und die hiermit verbundene Regenerierung der Matrix erfolgt, wie in Beispiel 1 beschrieben, mit 2 M NaCl-Lösung in PBS, pH 7.4. Die Eiweißkonzentration wurde kontinuierlich mit einem UV-Durchflußphotometer verfolgt. Die Ergebnisse entsprachen den in Beispiel 1 dargestellten Resultaten.

Beispiel 3: Die mit Chlorameisensäureestern aktivierte Perlzellulose (Aktivierungsgrad 63 µmol/ml) wird zunächst stufenweise in das wäßrige Milieu überführt, da die Perlzellulose mit Dioxan versetzt ist. Hierzu wird der Träger mit einem hinreichend großen Überschuß (50 ml Waschlösung/ml Träger, Dioxan-Wasser-Gemischen folgender Abstufungen unter Rühren gewaschen: 90/10; 70/30; 10/90; 0/100. Die Gesamtdauer des Waschprozesses sollte nicht länger als 10 - 15 Minuten betragen, um Hydrolyseverluste an aktiven Gruppen zu vermeiden. Danach wird der Träger mit dem Protein C1q (1 mg/ml) in PBS, pH 7.4, 10 mM EDTA enthaltend, versetzt und 4 h bei Raumtemperatur unter leichtem Schütteln inkubiert. Die Bestimmung des gebundenen Proteins C1q erfolgt durch Differenzmessung der Ausgangslösung bzw. des Überstandes nach vorheriger Dialyse bei 280 nm (E²⁸⁰_{1%} = 6,8) oder mit Hilfe der einfachen radialen Immunodiffusion. Abschließend wird der Träger mit 1 M Ethanolaminlösung in PBS, pH 7.4, versetzt, um eventuell noch freie reaktive Gruppen abzublocken. Schließlich wird der Träger mit einem 0,01 M Phosphat-Puffer, pH 7.4, der 0,05 M NaCl enthält, gewaschen. Dieses trägergebundene Protein C1q wird nunmehr zur Bindung der Immunkomplexe eingesetzt.
Hierzu wird die mit dem Protein C1q beladene Perlzellulose mit einem Patientenserum, das erhöhte Konzentrationen zirkulierender Immunkomplexe enthält (Patient mit Lupus erythematodes visceralis), im batch-Verfahren versetzt und 2 h bei Raumtemperatur inkubiert. Nach Entfernung des Trägers durch Dekantieren oder Zentrifugieren wird dieser im folgenden mit 0,01 M Phosphat-Puffer, pH 7.4, 0,05 M NaCl, gewaschen und anschließend mit 2 M NaCl in PBS, pH 7.4, regeneriert. Die Bestimmung der Immunkomplexe erfolgte wie in Beispiel 1 und 2 mit einem C1q-Festphasen-Enzymimmunoassay. Zur Ermittlung der unspezifischen Bindung an den Träger wurde die Eiweißkonzentration im spezifischen Eluat nach Lowry bestimmt sowie eine Immunelektrophorese durchgeführt. Eine mögliche Aktivierung des Komplementsystems wurde durch quantitative Bestimmung der Komplementkomponenten C1q und C3 verfolgt. Tabelle 1 zeigt ein charakteristisches Ergebnis einer derartigen Behandlung.

**Tabelle 1**

| | Zirkulierende Immunkomplexe (µg/ml Äquivalente aggr. IgG) | (%) |
|---|---|---|
| Patientenserum vor Behandlung | 90 | 100 |
| nach Behandlung | 62 | 69 |

Eine Aktivierung des Komplementsystems konnte nicht nachgewiesen werden.

Beispiel 4: An aktivierte Perlzellullose wird, wie in Beispiel 3 beschrieben, das humane Protein C1q gebunden. Diese Matrix wird in eine Chromatographiesäule (Durchmesser 4 mm) gefüllt und mit einem 0,01 M Phosphat-Puffer, pH 7.4, 0,05 M NaCl, gespült. Anschließend wird 1 ml Serum eines Patienten mit erhöhtem Immunkomplexspiegel aufgetragen und mit o.g. Puffer eluiert (Durchfluß 10 ml/h). Die Abspaltung der spezifisch gebundenen Immunkomplexe und die hiermit verbundene Regenerierung der Matrix erfolgte, wie in Beispiel 1 beschrieben, mit 2 M NaCl-Lösung in PBS, pH 7.4. Die Eiweißkonzentration wurde kontinuierlich mit einem UV-Durchflußphotometer verfolgt. Die Ergebnisse entsprachen den in Beispiel 3 dargestellten Resultaten.

Beispiel 5: Poröse Glaskugeln mit einer Porenweite von 135 nm werden nach Kochen mit 30%iger Salpetersäure und neutralem Waschen mit einer 2%igen Lösung des Silanhaftmittels NB 114 (Aminopropyltriäthoxysilan, VEB Chemiewerk Nünchritz) in Ethanol/Wasser (1:1), pH 3.5, für 4 h bei 60⁰C und 6 h bei 120°C aktiviert.
Nach Waschen mit Ethanol/Wasser sowie mehrmaligem Waschen mit PBS, pH 7.4, wird das so funktionalisierte Glas mit 2%igem Glutardialdehyd in 0,1 M Phosphat-Puffer, pH 6,8, für 2 Stunden bei 37⁰C inkubiert. Nach erneutem Waschen mit PBS wird das aktivierte Glas mit Protein C1q (500 µg/ml) in PBS, pH 7.4, die 10 mM EDTA enthält, versetzt und 4 h bei 37⁰C inkubiert. Nach weiteren Waschvorgängen werden die noch freien Aldehydgruppen mit 1 M Ethanolaminlösung blockiert. Nach wiederholtem Waschen mit 0,01 M Phosphat-Puffer, pH 7.4, der 0,05 M NaCl enthält, wird diese mit Protein C1q beladene Matrix zur Bindung der zirkulierenden Immunkomplexe eingesetzt. Hierzu werden die mit dem Protein C1q beladenen Glaskugeln mit einem Patientenserum, das erhöhte Konzentrationen an zirkulierenden Immunkomplexen enthält (Patient mit einem Lupus erythematodes visceralis), im batch-Verfahren versetzt und 2 Stunden bei Raumtemperatur inkubiert. Nach Entfernung des Trägers durch Dekantieren oder Zentrifugieren wird dieser im folgenden mit 0,01 M Phosphat-Puffer, pH 7.4, 0,05 M NaCl, gewaschen und anschließend mit 2 M NaCl in PBS, pH 7.4, regeneriert. Die Bestimmung der Immunkomplexe erfolgte wiederum mit einem C1q-Festphasen-Enzymimmunoassay. Zur Ermittlung der unspezifischen Bindungen an den Träger wurde die Eiweißkonzentration im spezifischen Eluat nach Lowry bestimmt sowie eine Immunelektrophorese durchgeführt. Tabelle 2 zeigt ein charakteristisches Ergebnis einer derartigen Behandlung.

**Tabelle 2**

| | Zirkulierende Immunkomplexe (µg/ml Äquivalente aggr. IgG) | (%) |
|---|---|---|
| Patientenserum vor Behandlung | 90 | 100 |
| nach Behandlung | 68 | 73 |

## Patentansprüche

1. Verwendung von an eine feste Phase kovalent über Brückenbildner gebundenem C1Q zur Herstellung eines Therapeutikums zur Entfernung pathogener Immunkomplexe aus dem Blutkreislauf.

2. Verwendung nach Anspruch 1, dadurch gekennzeichnet, daß als feste Phase Copolymere von 2-Hydroxyethylmethacrylat und Ethylenglykoldimethacrylat oder cellulosehaltige oder siliciumdioxidhaltige Materialien verwendet werden.

3. Verwendung nach Anspruch 1 oder 2, dadurch gekennzeichnet, daß die festen Phasen aus sphärischen, flächenförmigen oder faserförmigen Formkörpern bestehen oder aber als dünne Schichten, Filme oder Lacke auf anderen Trägern aufgebracht sind.

4. Verwendung nach einem der Ansprüche 1 bis 3, dadurch gekennzeichnet, daß als Trägermaterial sphärische Poly(hydroxyethylmethacrylat)-Copolymere zum Einsatz gelangen, deren Partikelgröße 10 bis 1000 µm beträgt und deren Porosität > 10 nm ist.

5. Verwendung nach einem der Ansprüche 1 bis 3, dadurch gekennzeichnet, daß das cellulosehaltige Material Perlcellulose ist, deren Partikelgröße 10 - 500 µm beträgt und deren Porosität > 10 nm ist.

6. Verwendung nach einem der Ansprüche 1 bis 3, dadurch gekennzeichnet, daß die siliciumdioxidhaltigen Trägermaterialien kugelförmig sind mit einer Partikelgröße von 1 - 1000 µm und einer Porenweite von 0.05 - 100 µm, oder als Fasern eingesetzt werden.

7. Verwendung nach einem der Ansprüche 1 bis 4, dadurch gekennzeichnet, daß als Brückenbildner Epichlorhydrin, CNBr, Carbodiimide oder andere homo- oder heterobifunktionelle Reagentien eingesetzt werden.

8. Verwendung nach einem der Ansprüche 1 bis 3 und 5, dadurch gekennzeichnet, daß als Brückenbildner Chlorameisensäureester, CNBr, Carbodiimde oder Glutaraldehyd eingesetzt werden.

9. Verwendung nach einem der Ansprüche 1 bis 4 und 6, dadurch gekennzeichnet, daß als Brückenbildner Silanhaftmittel, vorzugsweise unter Zusatz homo- oder heterobifunktioneller Reagentien, eingesetzt werden.

## Claims

1. Use of C1Q covalently bound to a solid phase by means of bridging substances for producing a therapeutic agent for removing pathogenic immune complexes from blood circulation.

2. Use according to claim 1, characterised in that copolymers of 2-hydroxyethylmethacrylate and ethylene glycol dimethacrylate or cellulose-containing or silicon dioxide-containing materials are used as the solid phase.

3. Use according to claim 1 or 2, characterised in that the solid phases consist of spherical, planiform or fibrous mouldings but are also applied to other substrates as thin layers, films or lacquers.

4. Use according to one of claims 1 to 3, characterised in that spherical poly(hydroxyethylmethacrylate) copolymers are used as substrate material, the particle size thereof being 10 to 1,000 µm and the porosity thereof being >10 nm.

5. Use according to one of claims 1 to 3, characterised in that the cellulose-containing material is bead cellulose, the particle size thereof being 10 - 500 µm and the porosity thereof being >10 nm.

6. Use according to one of claims 1 to 3, characterised in that the silicon dioxide-containing substrate materials are spherical having a particle size of 1 - 1,000 µm and a pore width of 0.05 - 100 µm, or are used as fibres.

7. Use according to one of claims 1 to 4, characterized in that epichlorohydrin, CNBr, carbodiimides or other homobifunctional or heterobifunctional reagents are used as bridging substances.

8. Use according to one of claims 1 to 3 and 5, characterised in that chloroformate, CNBr, carbodiimides or glutaraldehyde are used as bridging substances.

9. Use according to one of claims 1 to 4 and 6, characterised in that silane adhesives, preferably with addition of homobifunctional or heterobifunctional reagents, are used as bridging substances.

## Revendications

1. Utilisation d'un C1Q, fixé de façon covalente à une phase solide par l'intermédiaire de formateurs de ponts, pour la préparation d'un thérapeutique pour l'élimination des complexes immunogènes pathogènes hors du circuit sanguin.

2. Utilisation selon la revendication 1, caractérisée en ce que l'on utilise comme phase solide des copolymères de 2-hydroxyéthylméthacrylate et d'éthylèneglycoldiméthacrylate ou des matériaux présentant une teneur en cellulose ou en dioxyde de silicium.

3. Utilisation selon la revendication 1 ou 2, caractérisée en ce que les phases solides sont composées de corps façonnés, se présentant sous forme sphérique, plane ou fibreuse, ou bien comme couches minces, films ou vernis, appliqués sur d'autres supports.

4. Utilisation selon l'une des revendications 1 à 3, caractérisée en ce que l'on utilise comme matériau support un copolymère sphérique de poly(hydroxyéthylméthacrylate), dont la taille des particules va de 10 à 1000 µm et dont la porosité est supérieure à 10 nm.

5. Utilisation selon l'une des revendications 1 à 3, caractérisée en ce que le matériau présentant une teneur en cellulose est une Perlcellulose dont la taille de particules va de 10 à 500 µm et dont la porosité est supérieure à 10 nm.

6. Utilisation selon l'une des revendications 1 à 3, caractérisée en ce que les matériaux supports présentant une teneur en dioxyde de silicium se présentent sous forme sphérique, avec une taille de particules allant de 1 à 1000 µm et une largeur de pores allant de 0,05 à 100 µm ou ou qu'ils sont utilisés sous forme de fibres.

7. Utilisation selon l'une des revendications 1 à 4, caractérisée en ce que l'on utilise comme formateurs de ponts de l'épichlorhydrine, CNBr, un carbodiimide ou d'autres réactifs homo- ou hétérobifonctionnels.

8. Utilisation selon l'une des revendications 1 à 3 et 5, caractérisée en ce que l'on utilise comme formateurs de ponts un chloroester d'acide formique, CNBr, un carbodiimide, ou un glutaraldéhyde.

9. Utilisation selon l'une des revendications 1 à 4 et 6, caractérisée en ce que l'on utilise comme formateurs de ponts un agent adhésif au silane, de préférence avec addition de réactifs homo- ou hétérobifonctionnels.
